(19) Europäisches Patentamt
European Patent Office
Office européen des brevets

(11) Publication number: 0 405 599 A2

(12) EUROPEAN PATENT APPLICATION

(21) Application number: 90112494.1

(51) Int. Cl.5: C07F 7/08, C07C 19/08

(22) Date of filing: 29.06.90

A request for addition of pages 17a and 17b to the originally filed description has been filed pursuant to Rule 88 EPC. A decision on the request will be taken during the proceedings before the Examining Division (Guidelines for Examination in the EPO, A-V, 2.2).

(30) Priority: 30.06.89 US 373393

(43) Date of publication of application:
02.01.91 Bulletin 91/01

(84) Designated Contracting States:
DE FR GB IT

(71) Applicant: DOW CORNING CORPORATION
P.O. Box 1767
Midland Michigan 48686-0994(US)

(72) Inventor: Caporiccio, Gerardo
2026 Abbott Road, Apt. K-8
Midland, Michigan(US)

(74) Representative: Spott, Gottfried, Dr. et al
Patentanwälte Spott und Puschmann
Sendlinger-Tor-Platz 11
D-8000 München 2(DE)

# (54) Chemically inert fluorinated organosilicon compounds and fluorocarbon telomers for preparing same.

(57) Chemically inert fluorinated organosilicon compounds for use as, e.g., lubricants are described corresponding to the formulae $R^1{}_4Si$ or $R^2{}_3Si(R^3SiR^4{}_2)_zR^2$, where z is from 0 to 4, inclusive, at least three of the $R^1$ radicals, at least two of the terminal $R^2$ radicals and preferably one of the non-terminal $R^2$ radicals are derived from (a) monovalent hydrocarbyl-terminated telomers of chlorotrifluoroethylene or (b) monovalent hydrocarbyl-terminated cotelomers of chlorotrifluoroethylene and either hexafluoropropene or tetrafluoroethylene and $R^3$ represents a hydrocarbyl-terminated divalent telomer or cotelomer.

EP 0 405 599 A2

# CHEMICALLY INERT FLUORINATED ORGANOS ILICON COMPOUNDS AND FLUOROCARBON TELOMERS FOR PREPARING SAME

This invention relates to novel organosilicon compounds and intermediates for preparing these compounds. More particularly, this invention relates to chemically inert fluorinated organosilicon compounds wherein the majority of the organic groups bonded to silicon are formed by the reaction of halosilanes with novel telomers or cotelomers derived from a specified group of fluoroolefins. The organosilicon compounds are characterized by their chemical inertness.

The properties of certain tetraalkylsilanes make them useful as hydraulic fluids. If the silicon atom of these silanes is bonded to at least two different alkyl groups that preferably contain from 4 to 12 carbon atoms, it is possible to achieve the desired levels of viscosity and volatility, a fair level of thermal resistance and a high flash point. The disadvantage of these silanes is their flammability and the necessity to add various modifiers, including anti-wear additives for improving lubricity, antioxidants for higher oxidative stability, anti-corrosion agents to reduce damage to metal surfaces placed in contact with the silane in the presence of water or aqueous solutions of electrolytes.

The use of fluorinated substituents on organic polymers to improve both the thermal and chemical resistance of the polymer, in addition to providing anti-wear, optical and electrical insulating properties to the polymers, is well known.

An objective of this invention is to combine the desirable properties of both tetraalkylsilanes and organic polymers containing the aforementioned fluorinated substituents in a new class of materials characterized not only by the desirable properties of the fluorocarbon compounds but also by the favorable thermorheological properties provided by the large radius of the silicon atom.

The objectives of this invention are achieved by providing 1) novel fluorinated organosilicon compounds wherein the majority of the organic groups bonded to silicon are derived from hydrocarbyl-terminated telomers and/or cotelomers of selected fluoroolefins that are in themselves novel materials and 2) methods for preparing these novel telomers, cotelomers and organosilicon compounds.

This invention provides fluorinated organosilicon compounds corresponding to the formulae:

(1) $R^1{}_4Si$ or

(2) $R^2{}_3Si(R^3SiR^4{}_2)_zR^3SiR^2{}_3$

where at least three of the $R^1$ radicals, at least two of the $R^2$ radicals on each silicon atom and at least one of the $R^4$ radicals on each silicon atom are hydrocarbyl-terminated monovalent telomers and cotelomers of fluorinated olefins and any remaining $R^1$, $R^2$ and $R^4$ radicals are selected from the group consisting of alkyl radicals containing from 1 to 4 carbon atoms, fluoroalkyl radicals of the general formula $R'(CH_2)_y$-, phenyl and perfluoroalkyl-substituted phenyl, where $R'$ represents a perfluoroalkyl radical containing from 1 to 4 carbon atoms and $y$ is 2, 3 or 4;

$R^3$ represents a hydrocarbyl-terminated divalent telomer or cotelomer;

the value of $z$ is from 1 to 4, inclusive;

said hydrocarbyl-terminated monovalent telomer is represented by the formula:

(3) $R_f(C_2ClF_3)_n\text{-}CF_2CF(R^5)\text{-}C_mH_{2m}$-,

said hydrocarbyl-terminated monovalent cotelomer is represented by the formula

(4) $R_f(C_2ClF_3)_r\text{-}(C_pF_{2p})_q\text{-}CF_2CF(R^5)\text{-}C_mH_{2m}$-,

said hydrocarbyl-terminated divalent telomer is represented by the formula

(5) $-C_mH_{2m}\text{-}(R^5)CFCF_2\text{-}C_2ClF_3)_n\text{-}R_f'\text{-}(C_2ClF_3)_n\text{-}CF_2CF(R^5)\text{-}C_mH_{2m}$-

and said hydrocarbyl-terminated divalent cotelomer is represented by the formula

(6) $-C_mH_{2m}\text{-}(R^5)CFCF_2\text{-}(C_pF_{2p})_q(C_2ClF_3)_r\text{-}R_f'\text{-}(C_2ClF_3)_r\text{-}(C_pF_{2p})_qCF_2CF(R^5)\text{-}C_mH_{2m}$-

where the repeating units of said monovalent and divalent cotelomers are distributed randomly or sequentially;

$R_f$ represents a perfluoroalkyl radical containing from 1 to 4 carbon atoms,

$R_f'$ represents a perfluoroalkylene radical containing from 2 to 6 carbon atoms,

$R^5$ is fluorine or trifluoromethyl, the value of $m$ is 2, 3 or 4 with the proviso that $-C_mH_{2m}$- represents a linear radical;

the value of $n$ is from 1 to 20, inclusive, the value of $p$ is 2 or 3, $r$ and $q$ are each at least 1, the value of $r + q$ is from 2 to 20, inclusive, and the value of $r / q$ is from 2 to 10, inclusive.

Hydrocarbyl-terminated telomers and cotelomers represented by the foregoing formulae 3 through 6 where the terminal group $-C_mH_{2m}$- is replaced with $-C_{(m-1)}H_{(2m-3)}=CH_2$ or $-C_mH_{2m}X$, where X represents bromine or iodine, are novel compounds and as such constitute part of the present invention.

The silicon atoms of the present fluorinated organosilicon compounds are bonded by means of the non-halogenated linear alkylene radical $-C_mH_{2m}-$ containing 2, 3 or 4 carbon atoms to telomers of chlorotrifluoroethylene or to cotelomers of chlorotrifluoroethylene with either tetrafluoroethylene or hexafluoropropene.

One of the valences of silicon present in the preceding formula (1) and of the terminal silicon atoms in the preceding formula (2) when z is more than 1 can be satisfied by aryl, perfluoroalkyl substituted phenyl, alkyl and/or monovalent fluorinated alkyl radicals corresponding to the formula $-C_sH_{2s}R^6$ and where $R^6$ represents a perfluoroalkyl radical containing from 1 to 4 carbon atoms and the value of s is 2, 3 or 4.

The organosilicon compounds of this invention exhibit very low surface energy and a high resistance to heat and to chemically induced degradation. These properties make the present compounds particularly useful as lubricants and anti-wear materials, hydraulic fluids, bases for grease barrier and release materials. The present compounds also exhibit excellent optical properties, particularly low indices of refraction and provide high levels of electrical insulation.

The present compounds do not contain any chemically reactive groups in their structure and are therefore very chemically inert.

The present telomers and cotelomers are prepared from chlorotrifluoroethylene alone or in combination with either tetrafluoroethylene or hexafluoropropene.

Care must be exercised during preparation of the telomer or cotelomer and during the subsequent chain termination step to avoid the presence of a chlorine atom on each of two adjacent carbon atoms, i.e. the sequence =CCl-CCl= or the presence of the sequence =HC-CCl=. This precaution will avoid dehalogenation or dehydrohalogenation at high temperatures in the presence of metals and/or oxygen and/or catalysts, which may decrease the stability of the present organosilicon compounds during use.

When cotelomers are prepared, the chlorotrifluoroethylene and perfluorinated olefin must be polymerized in the proper sequence if the final organosilicon compound is to exhibit the high levels of chemical inertness and other properties required for critical conditions of use at high temperature and aggressive environments.

A preferred method for preparing the telomers and cotelomers of this invention is by a radical initiated telomerization of chlorotrifluoroethylene (CTFE) alone or in combination with either tetrafluoroethylene or one of the isomeric hexafluoropropenes. When these combinations containing CTFE are used the two olefins are either reacted as a mixture or the CTFE is reacted first, followed by subsequent addition of either tetrafluoroethylene or hexafluoropropene.

When chlorotrifluoroethylene is homotelomerized, care should be taken to avoid the sequence =CCl-CCl= in the final telomer.

Telomerization of the fluoroolefin(s) can be initiated by bromo- or iodo-substituted telogens represented by the formula $R_fX$ or $XR_f'X$, where $R_f$ and $R_f'$ are as previously defined in this specification and X is bromine or iodine, preferably iodine. Suitable telogens include but are not limited to $CF_3I$, $C_2F_5I$, n- or iso-$C_3F_7I$, n-$C_4F_9I$, $C_2F_5Br$, $CF_3CFBrCF_2BR$, $CF_3CFICF_2I$, $CF_2BrCFClBr$, $CF_2ICF_2I$ and $I(C_2F_4)_nI$, where the value of n is 1, 2 or 3.

To achieve the linear structure and the absence of significant crosslinking that characterizes the present organosilicon compounds, the synthesis of these compounds must be properly conducted with respect to the types and sequence of reactions leading to reaction of the tri- or tetrahalosilane with the appropriately terminated telomer or cotelomer.

The reaction between the telogen and the fluorinated olefin(s) is initiated by free radicals that can be generated by heating, preferably in the presence of an organic peroxide. The (co)telomerization can also be initiated by exposure to radiation such as gamma-rays or ultra-violet light, redox systems that include mercury, copper or iron salts and amines or other reducing agents, metal carbonyls derived from elements in groups VI, VII and VIII of the Periodic Table of Elements, alkylated boron compounds and the addition of stoichiometric amounts of oxygen. Preferred catalysts/initiators for these reactions include ultraviolet light, benzoyl peroxide, di-t-butyl peroxide, t-butylperoxypivalate and divalent mercury compounds such as mercuric chloride.

The (co)telomerization can be conducted in the presence of organic solvents including but not limited to 1,1,2-trichlorotrifluoroethane, t-butyl alcohol, acetonitrile and mixtures thereof. Catalysts such as persulfate redox systems can also be included in aqueous reaction mixtures. The temperature of the (co)-telomerization reaction can range from ambient to 140° C. if the reaction is initiated by irradiation or by catalysts or from 140° to 220° C. if the reaction is thermally initiated.

The pressure under which the reaction is conducted can range from ambient up to about 40 atmospheres. As with other free radical reactions, oxygen should be excluded from the reaction mixture.

Because the repeating units of the present (co)telomers contain units derived from $C_2ClF_3$, to avoid

possible dehalogenation or dehydrohalogenation, the telomerization process, including selection of comonomers, and the chain terminating process must be conducted in a manner that will completely avoid or at least minimize the sequences =ClC-CCl= and avoid the sequence =ClC-CH=.

When chlorotrifluoroethylene is the only monomer, the telomerization should be conducted at temperatures not exceeding 100 °C. If it is desired to conduct the telomerization at a higher temperature, an amount of tetrafluoroethylene or an isomeric hexafluoropropene sufficient to avoid as much as possible a "head-to-head" configuration due to sequences of $C_2ClF_3$ units should be present in the reaction mixture.

The telomers and cotelomers prepared from the foregoing telogens containing one or two terminal iodine or bromine atoms are linked through a non-halogenated di-, tri-or tetramethylene radical to a silicon atom using methods described in the following section of the specification to prepare silanes represented by the foregoing formula (1) or polysilylalkanes represented by formula (2).

Because all of the present homotelomers and many or all of the present cotelomers contain the terminal groups -CFClBr or -CFClI, these homo- and cotelomers must be reacted with a perfluoroolefin such as $C_3F_6$ or $C_2F_4$ in a process referred to in the present specification as pre-end capping. This step is conveniently accomplished by heating a mixture of the perfluoroolefin and the homo- or cotelomer in a sealed reactor under autogenous pressure.

The product of the pre-endcapping reaction is then reacted with ethylene or other non-halogenated olefin to form $-CH_2CH_2X$ terminal group that characterizes one species of the present (co)telomers. In this formula, X represents the iodine or bromine atom(s) present in the initial telogen. The introduction of this terminal group can be achieved by combining the pre-endcapped telomer or cotelomer with a suitable catalyst, such as a mixture of an amine and cuprous salt such as the chloride in a suitable vessel for accommodating pressurized gaseous reactants. The reactor is then sealed and the non-halogenated olefin introduced while the contents of the reactor are heated to a temperature of between about 100 and 200 °C. The pressure in the reactor is preferably maintained at between about 10 and 20 atmospheres during the introduction of the non-halogenated olefin.

In accordance with one preferred method for converting the present fluorinated (co)telomers containing one or two terminal groups of $(XC_mH_{2m})$- structure, where X is bromine or iodine and m is 2, 3 or 4, to one of the present organosilicon compounds involves formation of an organometallic derivative such as a Grignard reactant, Grignard-copper reactant or an organolithium, organozine or organoaluminum compound. The organometallic derivative then reacted with a silane containing a total of at least three halogen atoms and/or alkoxy groups. The remaining atoms on the silane can be alkyl radicals containing from 1 to 4 carbon atoms, fluoroalkyl radicals of the general formula $R'(CH_2)_y$-, phenyl and perfluoroalkyl-substituted phenyl, where $R'$ represents a perfluoroalkyl radical containing from 1 to 4 carbon atoms and y is 2, 3 or 4.

The organometallic compound can be prepared prior to being combined with the halo- or alkoxysilane or can be formed in the presence of this silane.

A second method for reacting the silane with the (co)telomer involves first removing the terminal iodine or bromine atom(s) of the (co)telomer by a dehydrohalogenation reaction to form a carbon-to-carbon double bond at the terminal position(s). These double bonds are then reacted with a silicon-bonded hydrogen atom present on the silane. This reaction is catalyzed by organic peroxides or platinum-containing catalysts.

The silanes used to prepare the present organosilicon compounds can contain an alkyl radical as a substituent if this is the only fully hydrogenated radical present on the silane.

It will be understood that when preparing organosilicon compounds containing two or more silicon atoms per molecule the sequence of said reactions is adjusted to achieve the desired number of silicon atoms in the final polysilylalkane chain, which can contain from two to six silicon atoms.

In summary, the reactions for preparing the present fluorosilicon compounds from the present telomers or cotelomers, include but are not limited to:

1) $R_fX + p\ CTFE + q\ M'(optional) \xrightarrow[\text{Peroxide}]{\text{UV or}} R_f(CTFE)_p(M')_q\ X$ (A)

CTFE = Chlorotrifluoroethylene

M' = Tetrafluoroethylene or hexafluoropropene

2) $A + RCF{=}CF_2 \longrightarrow R_f(CTFE)_p(M')_q\ CF_2\text{-}CFRX$ (B)

$R = F$ or $CF_3$

3) $B + C_2H_4 \longrightarrow R_f(CTFE)_r(M'')_q\ CF_2 - CFRC_2H_4X$ (C)

---

4a) $C + Me \longrightarrow D \xrightarrow{\equiv SiY} R_f(CTFE)_r(M')_qCF_2\text{-}CFRC_2H_4Si\equiv$

Me = metal = Mg, Li, Zn, Al, Mg/Cu;
Y = Br, Cl, alkoxy

4b) $C + KOH \longrightarrow R_f(CTFE)_p(M')_q\ CF_2CFRCH{=}CH2 \xrightarrow[\text{Pt Catalyst}]{SiH\equiv}$
$R_f(CTFE)_p(M')_qCF_2\text{-}CFRC_2H_4Si\equiv$

Pt = Platinum or a platinum compound.

The fluorinated organosilicon compounds of this invention include but are not limited to those of the following general formulae:

a) $(R_f''C_2F_2CF(R^5)C_mH_{2m})_4Si$, where $R_f''$ represents a monovalent telomer of CTFE or a cotelomer of CTFE with either tetrafluoroethylene or hexafluoropropene and m is 2, 3 or 4, preferably 2 or 3;

b) $[R_f''CF_2CF_2C(R^5)C_mH_{2m}]_3SiC_mH_{2m}CF_2CF(R^5)R_f'''\text{-}CF_2CF(R^5)C_mH_{2m}Si[C_mH_{2m}CF_2CF(R^5)R_f'']_3$, where $R_f''$ and m are defined in (a), $R_f'''$ is a divalent telomer of CTFE or a cotelomer of CTFE with either tetrafluoroethylene or hexafluoropropene.

The random structure and molecular weight of the present silanes make them eminently suitable for use as lubricants and hydraulic fluids.

The present silanes and polysilylalkanes exhibit high levels of chemical and thermal resistance and lubricity for the final fluorinated organosilicon compound. The compounds also impart a property of low moisture absorption that is particularly favorable for the use of the final organosilicon compounds as nonflammable hydraulic fluids.

Additional properties of the present organosilicon compounds included but are not limited to:

- low values of surface energy (30 to 20 dynes/cm), that is reflected in their high wetting power as liquids and low friction coefficient
- high lubricity and high hydro-oleophobicity that moreover is able to protect the bonds between silicon and carbon from chemical attack when the aggressive compounds are in aqueous solution
- low dielectric constant and high resistivity and dielectric strength
- good optical properties due to a low refractive index resulting from high fluorine content of the present compounds
- high resistance to solvents and aggressive chemicals
- high resistance to oxygen also at high temperature
- high resistance to depolymerization
- non-flammability
- resistance to irradiation and to high energy particles

These properties allow the use of the present organosilicon compounds as high performance barrier and release materials or as insulating material and optical media, as lubricants, hydraulic fluids or as ingredients greases.

Without further elaboration it is believed that one skilled in the art, using the preceding description, can utilize the present invention to its fullest extent. The following examples are, therefore, to be construed as merely illustrative and not limitative in any way whatsoever, of the remainder of the disclosure or claims.

The following examples describe preferred embodiments of the present (co)telomers and the novel organosilicon compounds prepared using these (co)telomers and should therefore not be interpreted as limiting the scope of the invention described in the accompanying claims with respect to said (co)telomers and organosilicon compounds. Unless otherwise noted all parts and percentages in the examples are by weight and viscosities were measured at 25 ° C.

## Example 1

This example describes the preparation of an ethylene-terminated telomer of CTFE.

A glass Carius tube was charged with 332 parts of a mixture of $C_2F_5I$ and chlorotrifluoroethylene in a molar ratio of 5:1, respectively. Each of these compounds was distilled into the tube under vacuum, after which the tube was sealed and exposed to the radiation from a 100 watt ultraviolet lamp for 110 hours. The tube was then opened and the volatile portion allowed to evaporate.

The residual liquid (14 parts by weight) in the polymerization tube was analyzed using gas/liquid chromatography and found to contain 41% $C_2F_5I$, 36% $C_2F_5$-$C_2F_3ClI$ and 15% $C_2F_5$-$(C_2F_3Cl)_2I$.

After removing the C2F5I by distillation 7 parts of the residual liquid were placed in a glass tube together with 10.7 parts of hexafluoropropene, the tube sealed and the contents heated at a temperature of 200 ° C. for 16 hours. When analyzed using gas/liquid chromatography the resultant liquid exhibit a longer retention time than the initial reactant.

All of the product from the previous reaction was placed in a 75 cc capacity autoclave together with 0.0035 parts of cuprous chloride, 0.104 part of ethanolamine and 10 parts of t-butanol. The autoclave was then sealed, filled with ethylene to a pressure of 20 atmospheres and then heated at a temperature of 150 ° C. for 16 hours. The product was found to have a higher retention time on a gas/liquid chromatography than the reactant. The spectrum obtained from analysis of the product by $F_{19}$ nuclear magnetic resonance (NMR) was consistent with the structures $C_2F_5(C_2F_3Cl)_nC_3F_6C_2H_4I$, where n is 1 and 2.

Reaction of this hydrocarbyl-terminated telomer with magnesium to form a Grignard reagent and subsequent reaction of this reagent with methyltrichlorosilane in a molar ratio of at least 3:1 is expected to yield a silane corresponding to the formula $[C_2F_5(C_2F_3Cl)_nC_3F_6C_2H_4]_3SiH_3$, where n is 1 and 2.

## Example 2

This application describes the sequential cotelomer of chlorotrifluoroethylene and hexafluoropropene.

A glass polymerization tube was evacuated and then charged by reduced pressure distillation with trifluoromethyl iodide and chlorotrifluoroethylene in a molar ratio of 5:1, respectively. The tube was then sealed and exposed to the radiation from a 100 watt ultraviolet lamp for 4 days. The tube was located 5 cm. from and oriented parallel to the lamp. The tube was then opened, the gases vented and the residual liquid analyzed using gas/liquid chromatography. A comparison of the retention times with those of other telomers indicated the product to be composed of three telomers of the general formula $CF_3(CF_2CFCl)_nI$ where n was 1 and 2. The relative areas under the three major peaks of the chromatogram indicated a molar ratio for the n = 1, n = 2 and n = 3 telomers of 5.5:1.3:0.9, respectively.

A 250 cc capacity stainless steel autoclave was charged with the telomer prepared as described in the first part of this example. The autoclave was then sealed and hexafluoropropene was distilled in under reduced pressure. The amount of hexafluoropropene added was equivalent to a molar ratio of initial telomer to hexafluoropropene of 1:6. The autoclave was then heated at 20 ° C. and rocked for 86 hours. After the unreacted gases had been discharged the residual liquid was analyzed using gas/liquid chromatography, an SE column and a heating program of 15 ° C. per minute to a final temperature of 270 ° C. The longer retention times of the final products relative to the initial telomers indicated that all had reacted to form endcapped telomers. Analysis of the final products using 60 MHz NMR supported the proposed structure $CF_3[CF_2CFCl]_r[CF_2CF(CF_3)]_qI$ where r = 1, 2 and 3 and q = 2. This structure was further supported by isolating the n = 1 fraction using preparative liquid/gas chromatography and analysis of this fraction using $F_{19}$ NMR. The shifts assigned to the various groups were as follows:

| Shift | Group |
|---|---|
| -72.5<br>-78.9<br>-97.9 to -109.5 and -118.6 to -119.4<br>-129 to -134<br>-138 to -142 | $-CF_3(C_3F_6)$<br>$-CF_3$<br>$-CF_2-$<br>-CFCl-<br>t-CF from $C_3F_6$ |

There were no signals attributable to the $CF_2I$ group.

Reaction of this cotelomer with ethylene as described in Example 1 is expected to yield a hydrocarbyl-terminated cotelomer corresponding to the formula $CF_3[CF_2CFCl]_r[CF_2CF(CF_3)]_qCH_2CH_2I$. Reaction of this hydrocarbyl-terminated telomer with magnesium to form a Grignard reagent and subsequent reaction of this reagent with methyltrichlorosilane in a molar ration of at least 3:1 is expected to yield a silane corresponding to the formula $[C_2F_5(C_2F_3Cl)_r(C_3F_6)_qC_2H_4]_3SiCH_3$, where r is 1 and 2 and q is 2.

Example 3

Divalent cotelomers were prepared following the general procedure of Example 1 but starting the cotelomerization process using 59 parts of the telogen $IC_2F_4I$ (PCR, Inc.) and then introducing into the glass Carius tube 44 parts CTFE. The tube was then sealed and exposed to the radiation from a 1000 watt ultraviolet lamp for 115 hours while being heated at 115 °C.

After discharging the unreacted gases from the tube, the liquid residue was shown by $F_{19}$ NMR to be a telomer having a degree of polymerization of about 13.

Reaction of this telomer with hexafluoropropene and reaction of the resultant pre-endcapped telomer with ethylene as described in Example 1, followed by conversion of the resultant hydrocarbyl-terminated telomer to the corresponding Grignard reagent by reaction with magnesium and reaction of this reagent with $R_f''''SiCl$ where $R_f''''$ represents $C_2F_5(C_2F_3Cl)_2C_3F_6C_2H_4$- is expected to yield a disilylfluoroalkane represented by the formula

$[C_2F_5(C_2F_3Cl)_2C_3F_6C_2H_4Si]_2\ CH_2CH_2(C_2F_3Cl)_mC_2F_4(C_2F_3Cl)_nCH_2CH_2$

where the total number of repeating units derived from chlorotrifluoroethylene is about 13.

Example 4

Conversion of an Ethylene-Terminated Telomer to a Silane.

An ethylene terminated telomer was prepared using a procedure similar to that described in the preceding example 1, and corresponded to the formula (A).

$(C_2F_5(C_2F_3Cl)_mC_3F_6C_2H_6I$ (A)

where the average value of n was 4. Under a nitrogen atmosphere thirty parts of this telomer were added to a glass reactor equipped with a magnetic stirrer, reflux condenser. The reactor contained 50 parts of anhydrous ethyl ether and 1.7 parts of magensium metal. The resultant mixture was heated at the boiling point for 8 hours.

The product of this reaction mixture was filtered under nitrogen and the filtrate combined with 5.1 parts of silicon tetrachloride dissolved in 10 parts of ethyl ether. The resultant mixture was reacted at reflux temperature for 48 hours.

At the end of this time period the liquid portion of the reaction mixture was separated from the solid material by filtration and poured into a mixture of 100 parts of ethanol and 30 parts of a 40% solution of hydrofluoric acid. The mixture had been previously cooled to 0 °C.

The organic layer was washed with ethanol, separated and stirred at room temperature with 1 part of anhydrous sodium fluoride. The final product was analyzed using $Si_{29}$ nuclear magnetic resonance (NMR).

The spectrum contained broad peaks in the region of 12.85-16.31 ppm using tetramethylsilane (TMS) as the marker. This was attributed to the compound represented by formula (B).

$R_fC_2H_6)_3SiOC_2H5$ (B)

where $R_fC_2H_6$ represents the ethylene-terminated fluorinated telomer block corresponding to the preceding

formula A.

16.5 parts of product B were combined with 20 parts of ehtyl ether and the resultant solution added to a two-fold molar excess of 3,3,3-trifluoropropyl lithium, $CF_3CH_2CH_2Li$ dissolved in 90 parts of ethyl ether. The resultant mixture was reacted at -30 ° C for 16 hours.

At the end the mixture was poured into a cooled 5% aqueous sulfuric acid solution,then washed with water, dried over anhydrous magnesium sulfate, and concentrated at 100 ° C under a pressure of 1 mm Hg.

The final product, a liquid, was analyzed using $Si_{29}$ NMR. The maxima in the region 12.85-16.31 ppm observed for product B had disappeared.

The final product and the intermediates represented by the preceding formulae (A) and (B) were analyzed using gel permeation chromatography with Styrogel(R) columns exhibiting increasing porosities of 100, 500, 1000, and 10,000 Angstroms and a pressure of 560 psi. The resultant chromatograms demonstrated the expected increase retention time progressing from product A to product C. The retention times were equivalent to an average molecular weight of about 900 for (A), increasing to about 2400-2500 for (B) and (C).

The analytical data are consistent with a structure for the final organosilicon compound, product (C), of
$(C_2F_5(C_2F_3Cl)_nC_3F_6C_2H_4)_3SiC_2H_4CF_3$
where the average value of n is 4.

## Claims

1. A fluorinated organosilicon compound corresponding to the formulae $R^1{}_4Si$ or $R^2{}_3Si(R^3SiR^4{}_2)_zR_3SiR^2{}_3$ where at least three of the $R^1$ radicals, at least two of the $R^2$ radicals on each silicon atom and at least one of the $R^4$ radicals on each silicon atom are hydrocarbyl-terminated monovalent telomers and cotelomers of fluorinated olefins and any remaining $R^1$, $R^2$ and $R^4$ radicals are selected from the group consisting of alkyl radicals containing from 1 to 4 carbon atoms, fluoroalkyl radicals of the general formula $R'(CH_2)_y$-, phenyl and perfluoroalkyl-substituted phenyl, where $R'$ represents a perfluoroalkyl radical containing from 1 to 4 carbon atoms and y is 2, 3 or 4;
$R^3$ represents a hydrocarbyl-terminated divalent telomer or cotelomer;
the value of z is from 0 to 4, inclusive;
said hydrocarbyl-terminated monovalent telomer is represented by the formula $R_f(C_2ClF_3)_n$-$CF_2CF(R^5)$-$C_mH_{2m}$-,
said hydrocarbyl-terminated monovalent cotelomer is represented by the formula
$R_f(C_2ClF_3)_r$-$(C_pF_{2p})_q$-$CF_2CF(R^5)$-$C_mH_{2m}$-,
said hydrocarbyl-terminated divalent telomer is represented by the formula
-$C_mH_{2m}$-$(R^5)CFCF_2$-$(C_2ClF_3)_n$-$R_f'$-$(C_2ClF_3)_n$-$CF_2CF(R^5)$-$C_mH_{2m}$-
and said hydrocarbyl-terminated divalent cotelomer is represented by the formula
-$C_mH_{2m}$-$(R^5)CFCF_2C$-$(C_pF_{2p})_q(C_2ClF_3)_r$-$R_f'$-$(C_2ClF_3)_r$-$(C_pF_{2p})_q$-$CF_2CF(R^5)$-$C_mH_{2m}$-
where the repeating units of said monovalent and divalent cotelomers are distributed randomly or sequentially;
$R_f$ represents a perfluoroalkyl radical containing from 1 to 4 carbon atoms,
$R_f'$ represents a perfluoroalkylene radical containing from 2 to 6 carbon atoms,
$R^5$ is fluorine or trifluoromethyl, the value of m is 2, 3 or 4 with the proviso that -$C_mH_{2m}$- represents a linear radical;
the value of n is from 1 to 20, inclusive, the value of p is 2 or 3, r and q are each at least 1, the value of r + q is from 2 to 20, inclusive and the value of r / q is from 2 to 10, inclusive.

2. A hydrocarbyl-terminated telomer of chlorotrifluoroethylene represented by the formula
$R_f(C_2ClF_3)_n$-$CF_2CF(R^5)$-$C_mH_{2m}X$ or
$XC_mH_{2m}$-$(R^5)CFCF_2$-$(C_2ClF_3)_n$-$Rf'$-$(C_2ClF_3)_n$-$CF_2CF(R^5)$-$C_mH_{2m}X$
where $R_f$ represents a perfluoroalkyl radical containing from 1 to 4 carbon atoms, $R_f'$ represents a perfluoroalkylene radical containing from 2 to 6 carbon atoms, $R^5$ is fluorine or trifluoromethyl, X is bromine or iodine, the value of m is 2, 3 or 4 with the proviso that -$C_mH_{2m}$- represents a linear radical and the value of n is from 1 to 20, inclusive.